(19) Europäisches Patentamt
European Patent Office
Office européen des brevets

(11) **EP 4 578 473 A1**

(12) **EUROPEAN PATENT APPLICATION**

(43) Date of publication:
**02.07.2025 Bulletin 2025/27**

(21) Application number: **24222970.6**

(22) Date of filing: **23.12.2024**

(51) International Patent Classification (IPC):
**A61M 1/36** (2006.01)   **B04B 5/04** (2006.01)
**B04B 13/00** (2006.01)

(52) Cooperative Patent Classification (CPC):
**A61M 1/36224; A61M 1/36225; A61M 1/3696;**
**B04B 5/0442; B04B 13/00;** A61M 2205/331;
A61M 2205/3313; B04B 2005/0492; B04B 2013/006

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **27.12.2023  US 202363615000 P**

(71) Applicant: **Fenwal, Inc.**
**Lake Zurich, IL 60047 (US)**

(72) Inventors:
• **KUSTERS, Benjamin E.**
**Lake Zurich, 60047 (US)**
• **MIN, Kyungyoon**
**Lake Zurich, 60047 (US)**

(74) Representative: **Maikowski & Ninnemann**
**Patentanwälte Partnerschaft mbB**
**Postfach 15 09 20**
**10671 Berlin (DE)**

(54) **METHOD FOR DETERMINING COMPLETION OF PLATELET COLLECTION PROCEDURE**

(57) An automated blood processing device includes one or more optical sensors configured to detect intensity of at least a portion of light through a fluid having a concentration of platelets and providing signals indicative of the intensity, and a controller configured to receive the signals from the at least one optical sensor. The controller is configured to determine an instantaneous concentration measurement of platelets within the fluid based on the received signals. In a method for determin- ing completion of a platelet collection procedure, an instantaneous platelet yield is determined based on the instantaneous platelet concentration and a flow rate, and a total platelet yield is determined based on a sum of the instantaneous platelet yields. The method may further include comparing the total platelet yield to a target platelet yield, and ending the procedure if the total platelet yield is greater than the target platelet yield.

FIG. 1

EP 4 578 473 A1

## Description

Background

Field of the Disclosure

The disclosure relates to an automated blood separation or processing device and more particularly, to a method for determining a platelet collection yield using optical-based measurements in a platelet collection procedure.

Description of Related Art

Various blood processing systems now make it possible to collect particular blood constituents, instead of whole blood, from a blood source. Typically, in such systems, whole blood is drawn from a blood source, the particular blood component or constituent is separated, removed, and collected, and the remaining blood constituents are returned to the blood source.

According to one approach, whole blood may be separated into cellular constituents (e.g., red blood cells) and non-cellular constituents (e.g., platelet-rich plasma) through centrifugation. The separated blood constituents may then be collected, directed back to the blood source (e.g., a donor) or further processed by the processing system. For example, the platelet-rich plasma may be further processed to separate platelets from the platelet-rich plasma, which produces platelet-poor plasma separated from a platelet concentrate. In such an example, the platelet concentrate may be collected as a platelet product and the platelet-poor plasma may be collected in another container as a plasma product or may be returned to the blood source.

According to a known platelet collection procedure, a platelet yield is calculated, and the procedure either proceeds or ends based on the calculated platelet yield. The platelet yield is calculated using complex mathematical modeling and depends on user input donor platelet pre-count measurement and precise volume tracking to enable the algorithm to estimate the concentration of platelets entering the system for collection. Although such calculations are effective, errors may occur that cause the devices to miss target platelet collection yields. Thus, platelet yields may be inconsistent and/or inaccurate.

Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, an automated blood processing system includes a blood processing device, a disposable fluid flow circuit arranged on the blood processing device, the disposable fluid flow circuit having one or more fluid paths configured to receive a fluid having a concentration of platelets, at least one optical sensor arranged relative to a fluid path of the one or more fluid paths, the optical sensor configured to provide one or more signals indicative of an intensity of at least a portion of light emitted into the fluid, and a controller configured to receive the signals from the at least one optical sensor, wherein the controller is configured to determine an instantaneous concentration measurement of platelets within the fluid based at least in part on the received signals.

In another aspect, an automated blood processing device includes one or more optical sensors configured to detect intensity of at least a portion of light through a fluid having a concentration of platelets and providing signals indicative of the intensity; and a controller configured to receive the signals from the at least one optical sensor, wherein the controller is configured to determine an instantaneous concentration measurement of platelets within the fluid based at least in part on the received signals.

In another aspect, a method of monitoring platelet yield in a platelet collection procedure using instantaneous concentration measurements, the method including determining an instantaneous platelet concentration based on optical sensor measurements taken at predetermined time intervals, determining a flow rate of the fluid containing the platelet concentration, determining the instantaneous platelet yield based on the instantaneous platelet concentration and the flow rate, and determining the total platelet yield based on a sum of the instantaneous platelet yields. The method may further include comparing the total platelet yield to a target platelet yield, and ending the procedure if the total platelet yield is greater than the target platelet yield. It is to be noted that the method may be conducted in an online modus (i.e. whole blood is withdrawn from any particular donor) or in an offline modus (i.e. whole blood is withdrawn from a container of previously collected blood of a donor).

Brief Description of the Drawings

Fig. 1 is a perspective view illustrating a blood processing device of a blood processing system, in accordance with an aspect of the present disclosure;
Fig. 2 is a schematic view showing an example disposable fluid flow circuit and portions of the blood processing device configured to interact with the disposable fluid flow circuit;
Fig. 3 is a schematic diagram showing an example of a cassette of the disposable fluid flow circuit;

Fig. 4 is a chart illustrating an instantaneous concentration of platelets determined using optical sensor measurements at predetermined time intervals;

Fig. 5 is a chart illustrating average flow rates of a fluid containing platelets between the predetermined time intervals of Fig. 4;

Fig. 6 is a chart illustrating an instantaneous platelet yield collected by the device between each sensor measurement interval;

Fig. 7 is a chart illustrating summed instantaneous platelet yield to determine total platelet yield collected by the device at any point in time through the platelet collection procedure;

Fig. 8 is a diagram illustrating an example of a method of performing a platelet collection procedure using instantaneous concentration measurements from one or more optical sensors; and

Fig. 9 is a diagram illustrating an example of a method of performing a platelet collection procedure using an average of instantaneous concentration measurements from one or more optical sensors.

Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

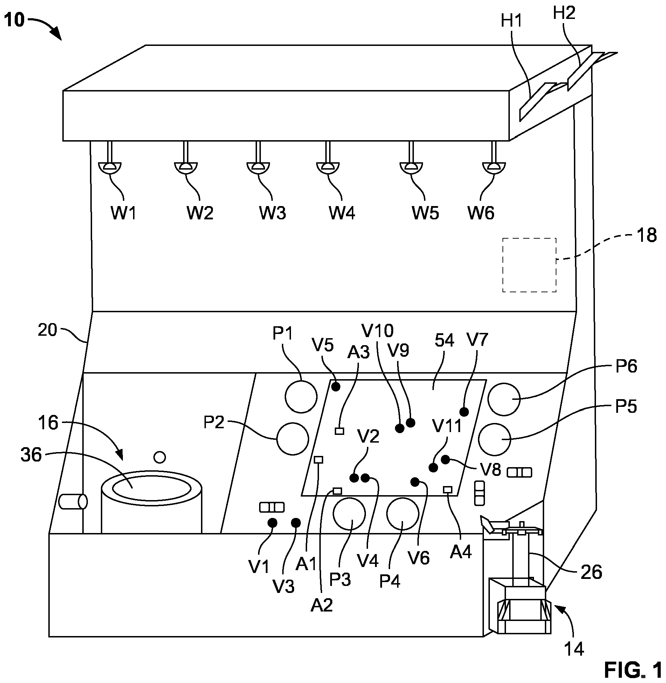

Figs. 1-3 generally show components of a blood or fluid separation or processing system 100 provided in accordance with the various examples described herein. While the system 100 may be referred to herein as a "blood processing system" or a "blood separation system" and examples will be given of various ways in which the system may be used to separate blood into its component parts, it should be understood that systems according to the present disclosure can be used for processing a variety of fluids, which may include bodily fluids and non-bodily fluids.

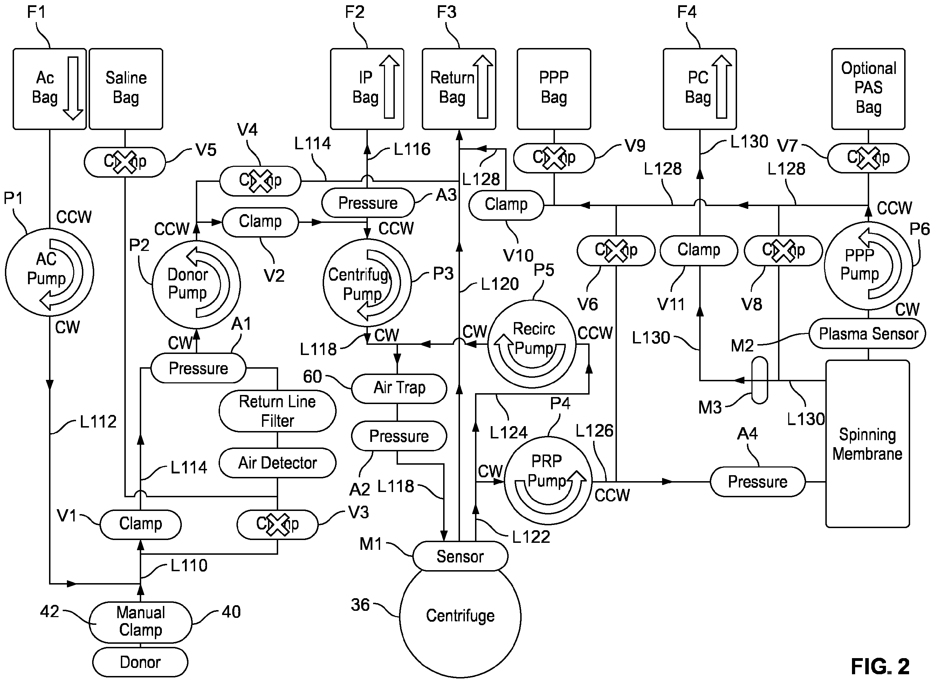

In general, the system includes two principal components, a durable and reusable blood processing device 10 (Fig. 1) and a disposable fluid flow circuit 12 (shown schematically in Fig. 2). In the illustrated example, the blood processing device 10 may include a spinning membrane separator drive unit 14, a centrifuge or centrifugal separator 16, additional components that control fluid flow through the disposable flow circuit 12, and a controller 18 (Fig. 1), which governs the operation of the other components of the blood separation device 10 to perform a blood processing procedure selected by the operator.

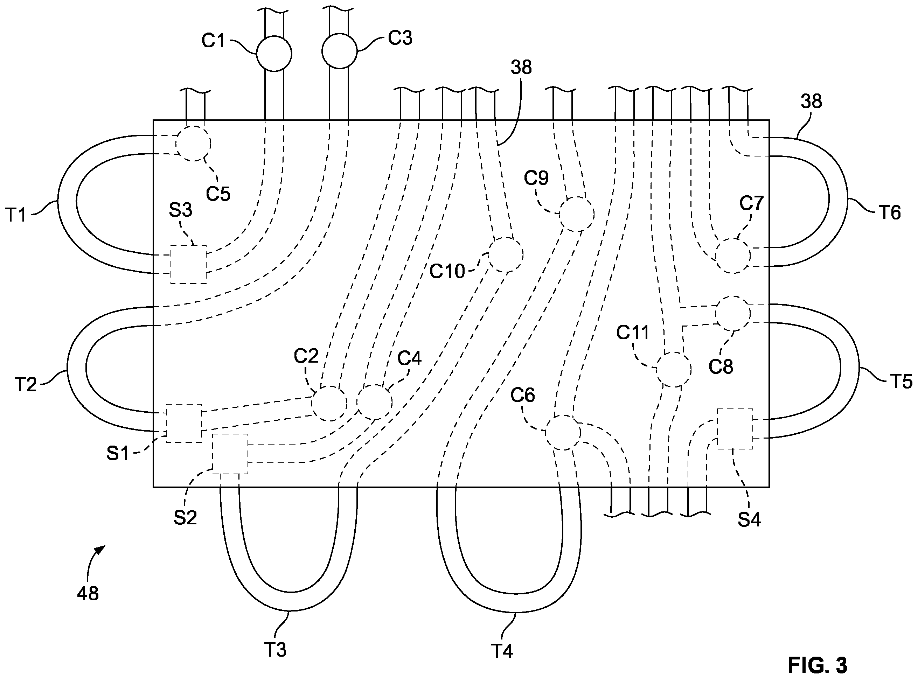

The disposable fluid flow circuit 12 may be selectively arranged on and removed from the blood processing device 10. The fluid flow circuit 12 may include one or more fluid containers F1-F4 (Fig. 2), one or more cassettes 48 (Fig. 3) accommodating a plurality of flow paths, a separation chamber 36 and flexible tubes 38 or tube segments configured to fluidically connect various components (e.g., the one or more fluid containers F1-F4, one or more cassettes 48 and/or separation chamber 36) to one another. Various components of the blood processing device 10 are configured to interact with corresponding components of the fluid flow circuit 12 to direct blood and/or blood components through the fluid flow circuit 12 according to the selected blood processing procedure, as described below.

According to the present examples, the blood processing system 100 may a perform one or more blood processing procedures, including one or more procedures in which platelets are separated from whole blood and then collected, generally referred to herein as a platelet collection procedure. Thus, the fluid flow circuit 12 may be configured with selected components and/or connections between the components and arranged on the blood separation device 10 in a predetermined manner to accommodate the separation and collection of platelets from whole blood. Various components of the blood separation device 10 may be operated to interact with corresponding components the fluid flow circuit 12 to direct fluid flow through circuit 12 according to parameters of the selected blood processing procedure.

The blood processing device 10 (Fig. 1) is configured as a durable item that is capable of long-term use. In the illustrated examples, the blood processing device 10 is embodied in a single housing or case into which the spinning membrane separator drive unit 14, centrifugal separator 16, controller 18 and other components may be incorporated.

The spinning membrane separator drive unit 14 is configured for accommodating a generally cylindrical membrane separator 26 which may be provided as part of the fluid flow circuit 12. Different membrane separators 26 may be selected depending on the selected blood processing procedure. The spinning membrane drive unit 14 is operable to spin one or more components of the spinning membrane separator 26 at a predetermined or selected speed and direction, depending on the selected blood processing procedure. In this manner, a fluid may be separated from the blood or blood component within the spinning membrane separator 26.

The centrifugal separator 16 may be provided in a compartment configured to receive, for example, the separation chamber 36 of the fluid flow circuit 12. Blood may be introduced into the separation chamber 36 and the centrifugal separator 16 may be operated to separate the blood into a layer of less dense components, such as platelet-rich plasma, and a layer of more dense components, such as pack red blood cells as a result of centrifugal forces as it rotates. The centrifugal separator 16 may be operated at different speeds for performing different blood processing procedures.

The blood processing device 10 includes various other components which may be selectively operated in conjunction with one or more of the spinning membrane separator drive unit 14, centrifugal separator 16 and the controller 18 in accordance with a selected or programmed blood processing procedure. For example, the blood processing device 10 may include one or more cassette stations 54 configured to accommodate a corresponding cassette 48 of the fluid flow circuit 12, a plurality of clamps or valves V1-V11, one or more pressure sensors A1-A4, a plurality of pumps P1-P6 and/or various other sensors M1-M3 for determining one or more properties of a fluid in fluid flow circuit 12.

The plurality of clamps or valves V1-V11 may be provided at the cassette stations 54 and are operable to selectively open and close the various flow paths formed on the cassettes 48. The pressure sensors A1-A4 may be positioned and configured to monitor pressure at selected locations in the system 100. The pressures sensors A1-A4 may transmit signals indicative of detected pressures to the controller 18.

The plurality of pumps P1-P6 are operable to cause fluid to flow through the fluid flow circuit 12. In the illustrated examples, the pumps P1-P6 are configured as peristaltic pumps, and each pump may be configured to engage a different tubing loop T1-T6 (Fig. 3) extending from a side surface of the cassette 48. The pumps P1-P6 may be selectively operated by the controller 18, in accordance with a selected blood processing procedure, to cause fluid to flow through a portion of the fluid flow circuit 12. Various other sensors may include a first optical sensor M1, a plasma sensor M2 and a second optical sensor M3.

Referring to Figs. 2 and 3, the disposable flow circuit 12, according to the present examples, generally includes the cassette 48 to which other components of the fluid flow circuit 12 are connected by the flexible tubing 38. Such other components may include the plurality of fluid containers F1-F4 (for holding blood, a separated blood component, an intravenous fluid or an additive solution), one or more blood source access device 40 (e.g., a phlebotomy needle or a connector for accessing blood within a fluid container), and the spinning membrane separator 26 and/or the centrifugal separation chamber 36.

The blood processing device 10 may further include a plurality of weight scales W1-W6 (Fig. 1, six are shown, but more or fewer may be provided), each of which may detect a weight of one or more fluid containers of the fluid flow circuit 12. The containers are configured to receive blood components separated during processing or intravenous fluids or additive fluids. Each weight scale W1-W6 transmits to the controller 18 a signal that is indicative of the weight of the fluid within the associated container to track the change of weight during the course of a procedure. In addition, the blood processing device 10 may include one or more hooks H1, H2 on which fluid containers may be supported.

Prior to performing a selected blood processing operation, the disposable flow circuit 12 may be arranged on the blood processing device 10 by positioning the cassette(s) 48 to interact with corresponding cassette stations 54, and the spinning membrane separator 26 to interact with the spinning membrane separator drive unit 14 and/or the centrifugal separation chamber 36 to interact with the centrifugal separator 16.

Referring to Figs. 2 and 3, with the cassette 48 positioned at the corresponding cassette station 54, the pumps P1-P6 are configured to interact with corresponding tubing loops T1-T6 of the cassettes 48 and the clamps or valves V1-V11 are configured to interact with corresponding valve stations C1-C11 of the cassette 48. One or more of the containers F1, F2, F3... may also be arranged on a container support of the blood separation device 10. The selected blood processing procedure may then be performed by controlling the pumps P1-P6 to cause fluid to flow in the circuit 12 via interaction with tubing loops T1-T6, controlling the clamps or valves V1-V11 to selectively open or close flow paths of the cassette 48 via interaction with valve stations C1-C11 and controlling the spinning membrane separator drive unit 14 to spin the spinning membrane separator 26 to separate a fluid from the whole blood and/or controlling the centrifugal separator 16 to separate blood into, for example, low and high density blood components via interaction with the centrifugal separation chamber 36. The various components of the blood processing device 10 may be controlled to operate by the controller 18 according to the parameters of a selected or programmed blood processing operation. In addition, the fluid flow circuit 12 may be configured differently (e.g., adding or removing fluid containers F1, F2, F3...) depending on the blood processing operation to be performed.

With further reference to Fig. 2, the fluid flow circuit 12 is schematically shown in an example configuration for a platelet collection procedure in which platelets are separated from whole blood to be collected in a container. The diagram of Fig. 2 also indicates operations of various components of blood processing device 10 and flow path(s) in the circuit 12 resulting from interactions between the various components of the processing device 10 and the corresponding components of the fluid flow circuit 12. The illustrated example shows a draw phase of a platelet collection procedure performed by the present blood processing system 5.

According to one aspect of the present disclosure, the blood separation device 10 may be used to separate and collect platelets from whole blood. Referring to Fig. 2, an exemplary fluid flow circuit 12 is configured to receive blood for processing through a blood access device 40 connected to a blood source (e.g., a container or a donor). In the illustrated example, the blood access device 40 is a single device (e.g., a single needle) that draws blood from and returns a separated blood component to the same location.

In the illustrated draw phase (Fig. 2), the donor pump P2 is operated to draw blood into line L110 of the fluid flow circuit 12. A manual clamp 42 may be selectively operated to allow or prevent blood flow into line L110 from the blood source. Anticoagulant ("AC") pump P1 is operated to draw and anticoagulant ("AC") fluid from container F1 into line L112. The AC fluid may be added to the blood at the junction of lines L110 and L112. The blood (with added AC fluid) then flows in line L114 through open valve V1 associated with valve station C1, pressure sensor A1 associated with sensor station S1, donor pump P2, and open valve V2 associated with valve station C2 to a junction with line L116 and L118. Valve V3, V4 and V5, associated with respective valve stations C3, C4, C5, are closed, to direct the fluid through line L114 as indicated above.

At the junction of lines L114, L116 and L118, a portion of the fluid from line L114 is directed through line L116 to the in-process container F2 via pressure sensor A3 and associated sensing station S3. Another portion of the fluid from line L114 is directed through line L118 to the centrifugal separation chamber 36. The fluid in line L118 is directed through the centrifugal pump P3, air trap 60, pressure sensor A2 associated with sensing station S2, and optical sensor M1 before being received by the separation chamber 36.

The centrifugal separator 16 of the blood processing device 10 manipulates the centrifugal separation chamber 36 to separate the blood in the centrifugal separation chamber 36 into platelet-rich plasma ("PRP") and packed red blood cells. The packed red blood cells exit the centrifugal separation chamber 36 and are directed to the return container F3 vial line L120. The PRP is drawn out of the centrifugal separation chamber 36 into line L 122 by the combined operation of the PRP and recirculation pumps P4 and P5. The optical sensor M1 is arranged relative to line L122 and is configured to measure the concentration of platelets in the PRP as described further below.

The PRP travels through line L122 until reaching a junction with lines L124 and L126. The recirculation pump P5 is associated with line L124 and redirects a portion of the PRP through line L124 to a junction with line L118, where the portion of the PRP mixes with the blood in line L118 and is recirculated to the centrifugal separation chamber 36. By such an arrangement, the flow rate of the fluid entering the centrifugal separation chamber 36 is equal to the sum of the flow rates of the centrifuge pump P3 and the recirculation pump P5. As the PRP drawn out of the centrifugal separation chamber 36 into line L124 by the recirculation pump P5 is immediately added back into the centrifugal separation chamber 36, the bulk or net platelet-rich plasma flow rate out of the centrifugal separation chamber 36 is equal to the flow rate of the PRP pump P4.

Valves V6 and V7, associated with valve stations C6 and C7, respectively, are closed such that another portion of the PRP is directed from line L122 into line L126 and flows into the spinning membrane separator 26. As indicated above, the optical sensor M1 may detect the concentration of platelets in the PRP exiting the centrifugal separation chamber 36 prior to entering the spinning membrane separator 26, while the pressure sensor A4 associated with sensing station S4 may monitor the pressure of the spinning membrane separator 26. According to one example, the platelet concentration of the PRP may be measured by optical sensor M1 as described in U.S. Pat. Appl. No. 18/101,275, incorporated herein by reference. For example, the optical sensor M1 may include a light source configured and oriented to emit a light into a fluid (i.e., the platelet-rich plasma) in a vessel, for example, the flexible tubing of the fluid flow circuit 12. The optical sensor M1 may also include a light detector array comprising a plurality of light detectors that are configured to receive at least a portion of the light exiting the vessel. The controller 18 may be configured to receive signals from the light detector array (of optical sensor M1) indicative of an intensity of the at least a portion of the light received by each one of the plurality of light detectors in the array, and determine a concentration of the platelets in the platelet-rich plasma based at least in part on the signals.

The spinning membrane separator drive unit 14 of the blood processing device 10 manipulates the spinning membrane separator 26 to separate the PRP into platelet-poor plasma ("PPP") and platelet concentrate ("PC"). In addition to valves V6 and V7, valves V8 and V9, associated with valve stations C8 and C9, respectively, are closed and valve V10, associated with valve station C10, is open. The PPP pump P6 is operated to draw the PPP out of the spinning membrane separator 26 and into line L128. With valves V6, V7, V8 and V9 closed, and valve V10 open, operation of the PPP pump P6 directs the PPP through line L128, open valve V10, and into line L120 for collection in the return container F3. On the way to the return container F3, the PPP passes through plasma sensor M2, which may cooperate with the controller 18 to determine one or more characteristics of the plasma, such as the amount of cellular blood components in the plasma and/or whether the plasma is hemolytic and/or lipemic.

The PC is conveyed out of the spinning membrane separator 26 into line L130. There is no pump associated with line L130, so instead the flow rate at which the PC exits the spinning membrane separator 26 is equal to the difference between the flow rates of the PRP pump P4 and PPP pump P6. According to one example, another optical sensor, i.e., a second optical sensor M3 may be positioned along line L130 and configured to measure the platelet concentration in the PC. The second optical sensor M3 may measure the platelet concentration in line L130 in the same manner as the optical sensor M1 measures the platelet concentration of PRP in line L122, e.g., by providing signals to the controller 18 indicative of an intensity of at least a portion of the light received by each one of the plurality of the light detectors of the second optical sensor M3.

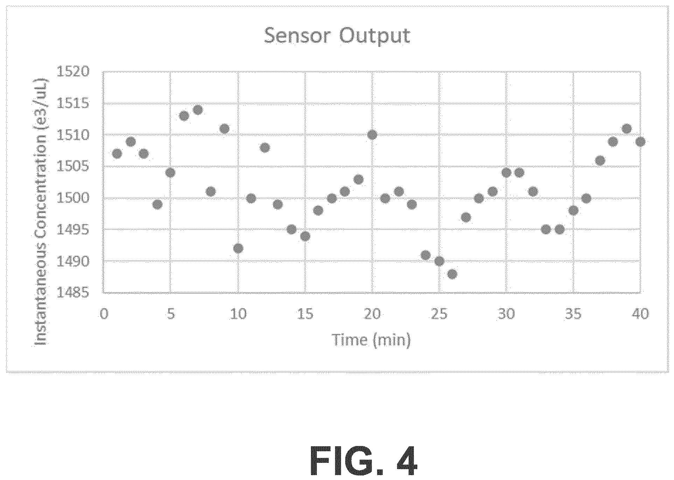

The valve V11 associated with valve station C11 along line L130 is open. Accordingly, the PC conveyed out of the spinning membrane separator 26 into the line L130 may be collected in the PC container F4. The platelet concentration measurements provided the first and second optical sensors M1, M3 in conjunction with the controller 18 may reflect an instantaneous concentration $C_{inst}$ measurement of the fluid present adjacent to the corresponding sensor M1, M3 at a single point in time. For example, as shown in the example of Fig. 4, the instantaneous concentration determined based at least in part on the sensor output, may be taken at 1-minute intervals. It will be appreciated, however, that such a time interval is provided as a non-limiting illustrative example, and that different time intervals may be used without departing from the present scope.

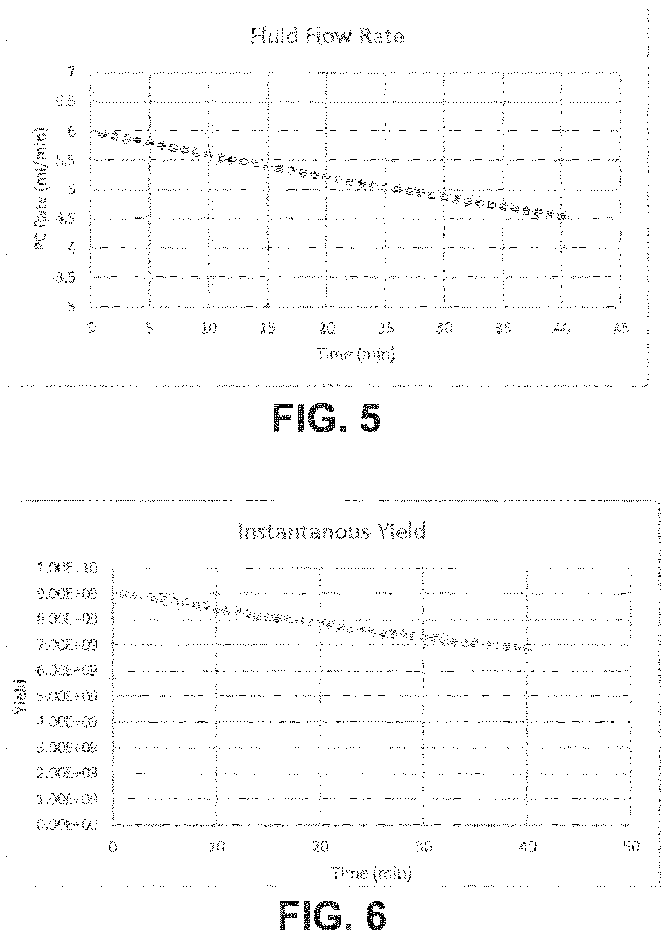

The total quantity (i.e., the yield) of the platelets is further based on the flow rate of the platelet containing fluid $Q_{PLTs}$ (i.e., the PRP or the PC) through the corresponding sensor M1, M3. The flow rate $Q_{PLTs}$ may be determined by the target pump rate, pump rotation feedback (tachs), weigh scale changes, or a combination of these or any other flow monitoring methods. The flow rates may be measured as instantaneous values at the same time points as the optical sensor measurement. As shown in Fig. 5, in some examples, the flow rates may be measured as average flow rates during the time interval between optical sensor measurements.

Referring now to Fig. 6, the instantaneous PLT yield $Yield_{Inst}$ collected by the device between each optical sensor M2, M4 measurement interval may be determined by multiplying the instantaneous concentration $C_{Inst}$ (Fig. 4) by the flow rate $Q_{PLTs}$ (Fig. 5). An average of the current instantaneous concentration $\underline{C_{Inst}}$ and the prior instantaneous concentration $C_{Inst-1}$ may be determined as follows:

$$Yield_{Inst} = C_{Inst} * Q_{PLTs}$$

where $C_{Inst}$ may be replaced by:

$$C_{Inst-Avg} = (C_{Inst} + CI_{nst-1}) / 2$$

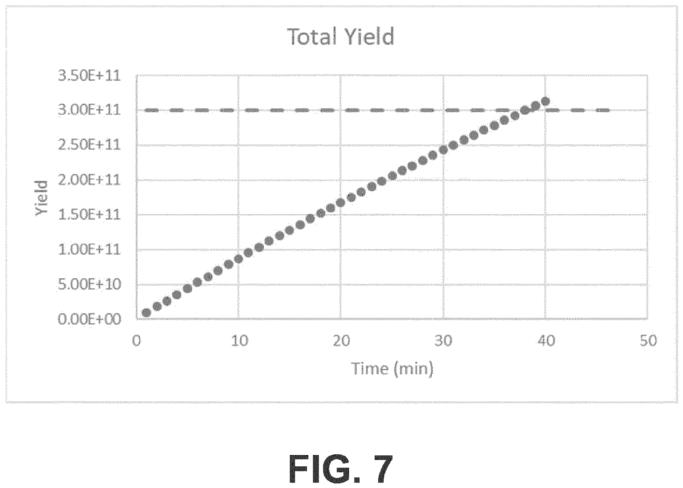

Referring to Fig. 7, the instantaneous platelet yield $Yield_{Inst}$ measurements may be summed to determine the total platelet yield $Yield_{Total}$ collected by the device at any point in time through a procedure. In some examples, the system may continue to collect platelets by continuing the platelet collection procedure until the total platelet yield $Yield_{Total}$ surpasses a target platelet yield $Yield_{Target}$, indicated by the dashed line at 3.00E+11 in Fig. 6. In the example of Fig. 7, the $Yield_{Target}$ may be 3.00E+11. However, such a yield target value is provided as a non-limiting illustrative example, and it will be appreciated that other yield target values may be used and are within the scope of the present disclosure. The $Yield_{Total}$ may be expressed as:

$$Yield_{Total} = \sum Yield_{Inst}$$

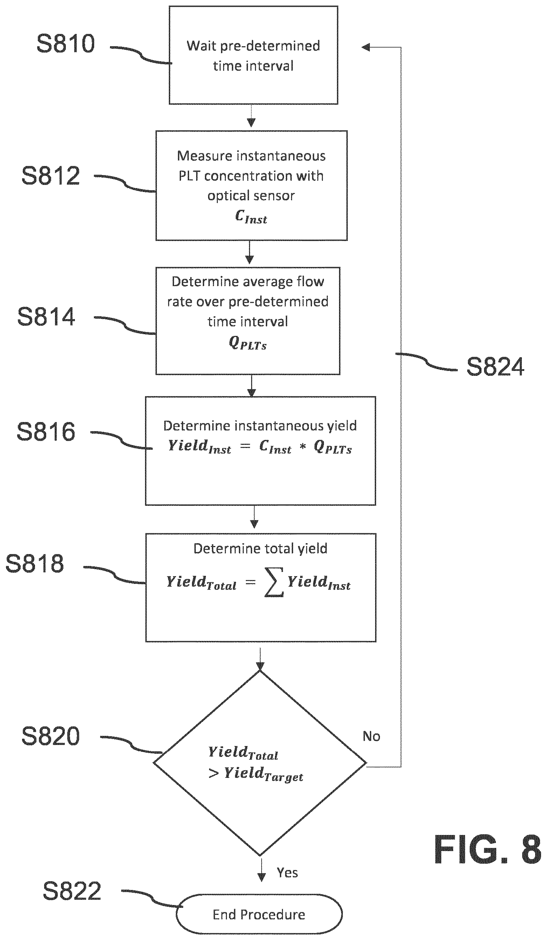

With reference to Fig. 8, a method of monitoring platelet yield in a platelet collection procedure using instantaneous concentration measurements may include, at S810, waiting a pre-determined time interval and at S812, measuring the instantaneous platelet concentration with an optical sensor M1, M3. The method may also include, at S814, determining an average flow rate over a predetermined time interval, at S816 the method may include determining the instantaneous yield, and at S818 the method may include determining the total yield. The system may be configured to continue performing the platelet collection procedure until a target yield has been collected. Accordingly, the method may further include, at S820, comparing the total yield to the target yield, and at S822, ending the procedure if the total yield is greater than the target yield. At S824, the method may be continued at S710 if the total yield is not greater than the target yield.

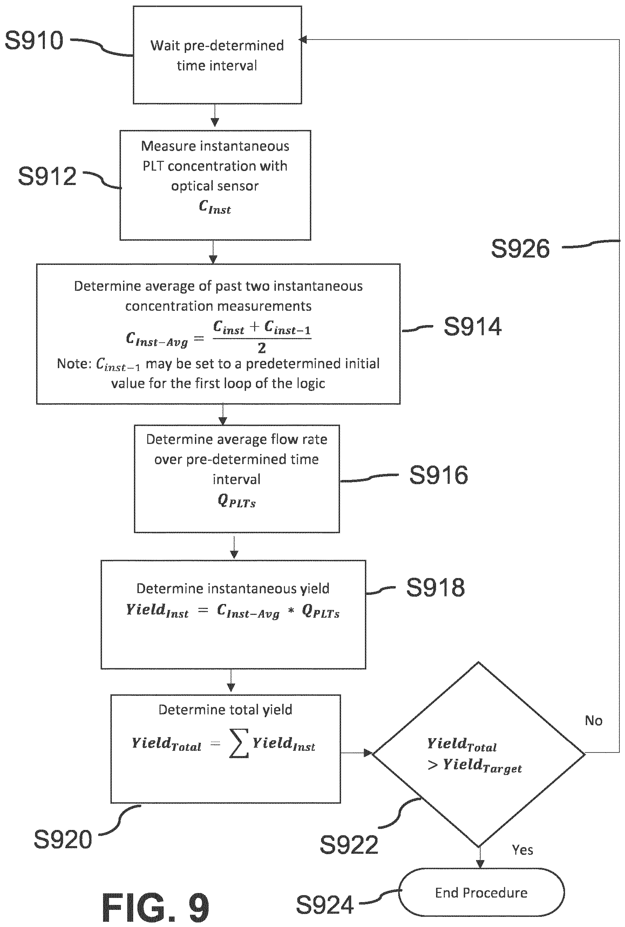

Referring now to Fig. 9, a method of monitoring platelet yield in a platelet collection procedure using an average of instantaneous concentration measurements may include, at S910, waiting a pre-determined time interval and at S912, measuring the instantaneous platelet concentration with an optical sensor M1, M3. The method may also include, at S914, determining an average of past two instantaneous concentration measurements. The value of $C_{inst-1}$ may be set to a predetermined initial value for the first loop. At S916, the method may include determining an average flow rate over a predetermined time interval, at S918 the method may include determining the instantaneous yield, and at S920 the method may include determining the total yield. The system may be configured to continue performing the platelet collection procedure until a target yield has been collected. Accordingly, the method may further include, at S922, comparing the total yield to the target yield, and at S924, ending the procedure if the total yield is greater than the target yield. At S926, the method may be continued at S910 if the total yield is not greater than the target yield.

Referring again to Fig. 1, the controller 18 is shown schematically as part of the blood processing device 10. The controller 18 is suitably configured and/or programmed to control operation of the blood processing device 10. In one example, the controller 18 comprises a main processing unit (MPU), which can comprise, e.g., a Pentium™ type microprocessor made by Intel Corporation, although other types of conventional microprocessors can be used. In one example, the controller 18 may be mounted inside the case 20, adjacent to or incorporated into an operator interface station (e.g., a touchscreen). In other examples, the controller 18 and operator interface station may be incorporated into a separate device that is connected (either physically, by a cable or the like, or wirelessly) to the blood processing device 10.

The microprocessor may be operatively coupled to one or more memory devices. The microprocessor may include one or more processors and/or other suitable circuitry configured to execute one or more program instructions, and in response to executing the program instructions, perform one or more functions according to the program instructions. The one or more memory devices may include any suitable data storage mechanism or system, such as a random access memory (RAM), read only memory (ROM), disk drive, solid state memory drive, optical disc drive and the like, including various combinations of such data storage mechanisms or systems. The one or more memory devices may also include, for example, a non-transitory computer-readable storage medium configured to store and allow access to the program instructions, code and/or other data.

The controller 18 is configured and/or programmed to execute at least one blood processing application but, more advantageously, is configured and/or programmed to execute a variety of different blood processing applications. For example, the controller 18 may be configured and/or programmed to carry out one or more of the following: a double unit red blood cell collection procedure, a plasma collection procedure, a plasma/red blood cell collection procedure, a red blood cell/platelet/plasma collection procedure, a platelet collection procedure, and a platelet/plasma collection procedure. In the present examples, the controller 18 is configured to execute a platelet collection procedure. Additional or alternative procedure applications can be included without departing from the scope of the present disclosure.

The controller 18 is operably connected to various components of the blood processing device 10 and may be configured to transmit a control signal to one or more of the components to control operations of that component. For example, the control signal provided by the controller 18 to a component to start or stop operations of the component (i.e., to turn the component on or off, or to energize and de-energize the component) and/or operate the component according to parameters associated with a selected blood processing procedure (e.g., speed or rotational speed, movements, movement direction (e.g., rotational direction) and/or duration. In the illustrated examples, the controller 18 may be connected to, and configured to operate one or more of the pumps P1-P6, the centrifugal separator 16, the spinning membrane separator drive unit 14, and/or one or more of the valves V1-V11.

The controller 18 may also be operably connected to one or more of the sensors A1-A4, M1-M3, of the blood processing device 10. In some examples, the controller 18 may be configured to receive signals and/or information from one or more of the sensors, wherein the signals or information may be indicative of a measured or detected parameter. The controller 18 may process the signal and/or information received from the one or more sensors and, in some examples, control operations of one or more components based, in part, on the received signals or information.

In carrying out a blood processing procedure, the controller 18 may be configured and/or programmed to operate component(s) of the blood processing device 10 to control one or more of the following tasks: drawing blood into a fluid flow circuit 12 mounted to the blood separation device 10, conveying blood through the fluid flow circuit 12 to a location for separation (i.e., into a spinning membrane separator 26 or centrifugal separation chamber 36 of the fluid flow circuit 12), separating the blood into two or more components as desired, and conveying the separated components into storage containers, to a second location for further separation (e.g., into whichever of the spinning membrane separator 26 and centrifugal separation chamber 36 that was not used in the initial separation stage), or to a recipient (which may be a donor from which the blood was originally drawn).

If provided, an operator interface station associated with the controller 18 allows the operator to view on a screen or display (in alpha-numeric format and/or as graphical images) information regarding the operation of the system. The operator interface station also allows the operator to select applications to be executed by the controller 18, as well as to change certain functions and performance criteria of the system. If configured as a touchscreen, the screen of the operator interface station can receive input from an operator via touch-activation. Otherwise, if the screen is not a touchscreen, then the operator interface station may receive input from an operator via a separate input device, such as a computer mouse or keyboard. It is also within the scope of the present disclosure for the operator interface station to receive input from both a touchscreen and a separate input device, such as a keypad.

Method steps and various functions described in the examples above may be performed and/or initiated by the controller 18.

Aspects

Aspect 1. An automated blood processing system includes a blood processing device, a disposable fluid flow circuit arranged on the blood processing device, the disposable fluid flow circuit having one or more fluid paths configured to receive a fluid having a concentration of platelets, and at least one optical sensor arranged relative to a fluid path of the one or more fluid paths, the optical sensor configured to provide one or more signals indicative of an intensity of at least a portion of light emitted into the fluid. The system further includes a controller configured to receive the signals from the at least one optical sensor, wherein the controller is configured to determine an instantaneous concentration measurement of platelets within the fluid based at least in part on the received signals.

Aspect 2. The automated blood processing system according to Aspect 1, wherein the controller is configured to determine the instantaneous concentration at predetermined time intervals.

Aspect 3. The automated blood processing system according to Aspect 1, wherein the controller is configured to determine a flow rate of the fluid containing the platelets through the optical sensor.

Aspect 4. The automated blood processing system according to Aspect 3, wherein the determined flow rate is an instantaneous flow rate measured at the same point in time as the optical sensor measurement.

Aspect 5. The automated blood processing system according to Aspect 3, wherein the determined flow rate is an average flow rate determined as an average flow rate in a time interval between optical sensor measurements.

Aspect 6. The automated blood processing system according to Aspect 3, wherein the controller is configured to determine an instantaneous platelet yield based at least in part on the instantaneous concentration on the determined flow rate of the fluid.

Aspect 7. The automated blood processing system according to Aspect 6, wherein the controller is configured to determine a total platelet yield collected by the blood processing device at any point in time, the total platelet yield based at least in part on a sum of the instantaneous platelet yields with respect to time.

Aspect 8. The automated blood processing system according to Aspect 7, wherein the controller is configured to compare the total platelet yield to a target platelet yield and control operations of the blood processing device depending the comparison.

Aspect 9. The automated blood processing system according to any of Aspects 1-8, wherein the determined platelet concentration is a concentration of platelets in platelet-rich plasma.

Aspect 10. The automated blood processing system according to any of Aspects 1-8, wherein the determined platelet concentrations is a concentration of platelets in platelet concentrate.

Aspect 11. An automated blood processing device comprising: one or more optical sensors configured to detect intensity of at least a portion of light through a fluid having a concentration of platelets and providing signals indicative of the intensity; and a controller configured to receive the signals from the at least one optical sensor, wherein the controller is configured to determine an instantaneous concentration measurement of platelets within the fluid based at least in part on the received signals.

Aspect 12. The automated blood processing device according to Aspect 11, wherein the at least one optical sensor includes a light source configured to emit a light into the fluid in the fluid path and a light detector array comprising a plurality of light detectors configured to receive the at least a portion of the light exiting the vessel, wherein the signals are indicative of the intensity of the at least a portion of the light received by each one of the light detectors in the light detector array.

Aspect 13. The automated blood processing device according to Aspect 11, wherein the controller is configured to determine a instantaneous platelet yield based at least in part on the instantaneous concentration of the platelets and a flow rate of the fluid containing the platelets through the at least one optical sensor.

Aspect 14. The automated blood processing device according to Aspect 13, wherein the controller is configured to determine a total platelet yield based at least in part a sum of the instantaneous platelet yields.

Aspect 15. The automated blood processing device according to

Aspect 14, wherein the controller is configured to compare the total platelet yield to a target platelet yield and control operations of one or more blood processing device components depending the comparison.

Aspect 16. A method of monitoring platelet yield in a platelet collection procedure using instantaneous concentration measurements, the method comprising: determining an instantaneous platelet concentration based on optical sensor measurements taken at predetermined time intervals; determining a flow rate of the fluid containing the platelet concentration; determining the instantaneous platelet yield based on the instantaneous platelet concentration and the flow rate; determining the total platelet yield based on a sum of the instantaneous platelet yields; comparing the total platelet yield to a target platelet yield; and ending the procedure if the total platelet yield is greater than the target platelet yield.

Aspect 17. The method according to Aspect 16, wherein the determined flow rate is an average flow rate at the optical sensor during the time interval between measurements.

Aspect 18. The method according to Aspect 16, wherein determining the instantaneous platelet concentration includes determining an average of the past two instantaneous platelet concentrations.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

**Claims**

1. An automated blood processing system comprising:

    a blood processing device;

a disposable fluid flow circuit arranged on the blood processing device, the disposable fluid flow circuit having one or more fluid paths configured to receive a fluid having a concentration of platelets;

at least one optical sensor arranged relative to a fluid path of the one or more fluid paths, the optical sensor configured to provide one or more signals indicative of an intensity of at least a portion of light emitted into the fluid; and a controller configured to receive the signals from the at least one optical sensor, wherein the controller is configured to determine an instantaneous concentration measurement of platelets within the fluid based at least in part on the received signals.

2. The automated blood processing system according to claim 1, wherein the controller is configured to determine the instantaneous concentration at predetermined time intervals.

3. The automated blood processing system according to claim 1, wherein the controller is configured to determine a flow rate of the fluid containing the platelets through the optical sensor.

4. The automated blood processing system according to claim 3, wherein the determined flow rate is an instantaneous flow rate measured at the same point in time as the optical sensor measurement or wherein the determined flow rate is an average flow rate determined as an average flow rate in a time interval between optical sensor measurements.

5. The automated blood processing system according to claim 3, wherein the controller is configured to determine an instantaneous platelet yield based at least in part on the instantaneous concentration and the determined flow rate of the fluid.

6. The automated blood processing system according to claim 5, wherein the controller is configured to determine a total platelet yield collected by the blood processing device at any point in time, the total platelet yield based at least in part on a sum of the instantaneous platelet yields with respect to time, preferably wherein the controller is configured to compare the total platelet yield to a target platelet yield and control operations of the blood processing device depending on the comparison.

7. The automated blood processing system according to any of claims 1-6, wherein the determined platelet concentration is a concentration of platelets in platelet-rich plasma or wherein the determined platelet concentrations is a concentration of platelets in platelet concentrate.

8. An automated blood processing device comprising:

one or more optical sensors configured to detect intensity of at least a portion of light through a fluid having a concentration of platelets and providing signals indicative of the intensity; and a controller configured to receive the signals from the at least one optical sensor, wherein the controller is configured to determine an instantaneous concentration measurement of platelets within the fluid based at least in part on the received signals.

9. The automated blood processing device according to claim 8, wherein the at least one optical sensor includes a light source configured to emit a light into the fluid in the fluid path and a light detector array comprising a plurality of light detectors configured to receive the at least a portion of the light exiting the vessel, wherein the signals are indicative of the intensity of the at least a portion of the light received by each one of the light detectors in the light detector array.

10. The automated blood processing device according to claim 8, wherein the controller is configured to determine a instantaneous platelet yield based at least in part on the instantaneous concentration of the platelets and a flow rate of the fluid containing the platelets through the at least one optical sensor.

11. The automated blood processing device according to claim 10, wherein the controller is configured to determine a total platelet yield based at least in part a sum of the instantaneous platelet yields.

12. The automated blood processing device according to claim 11, wherein the controller is configured to compare the total platelet yield to a target platelet yield and control operations of one or more blood processing device components depending the comparison.

13. A method of monitoring platelet yield in a platelet collection procedure using instantaneous concentration measurements, the method comprising:

determining an instantaneous platelet concentration based on optical sensor measurements taken at predetermined time intervals determining a flow rate of the fluid containing the platelet concentration; determining the instantaneous platelet yield based on the instantaneous platelet concentration and the flow rate; determining the total platelet yield based on a sum of the instantaneous platelet yields; comparing the total platelet yield to a target platelet yield; and
ending the procedure if the total platelet yield is greater than the target platelet yield.

14. The method according to claim 13, wherein the determined flow rate is an average flow rate at the optical sensor during the time interval between measurements.

15. The method according to claim 13, wherein determining the instantaneous platelet concentration includes determining an average of the past two instantaneous platelet concentrations.

FIG. 1

EP 4 578 473 A1

**FIG. 2**

FIG. 3

# FIG. 4

**FIG. 5**

**FIG. 6**

FIG. 7

S810 — Wait pre-determined time interval

S812 — Measure instantaneous PLT concentration with optical sensor $C_{Inst}$

S814 — Determine average flow rate over pre-determined time interval $Q_{PLTs}$

S816 — Determine instantaneous yield $Yield_{Inst} = C_{Inst} * Q_{PLTs}$

S818 — Determine total yield $Yield_{Total} = \sum Yield_{Inst}$

S820 — $Yield_{Total} > Yield_{Target}$

No

S824

Yes

S822 — End Procedure

**FIG. 8**

**S910** — Wait pre-determined time interval

**S912** — Measure instantaneous PLT concentration with optical sensor $C_{Inst}$

**S914** — Determine average of past two instantaneous concentration measurements
$$C_{Inst-Avg} = \frac{C_{inst} + C_{inst-1}}{2}$$
Note: $C_{inst-1}$ may be set to a predetermined initial value for the first loop of the logic

**S916** — Determine average flow rate over pre-determined time interval $Q_{PLTs}$

**S918** — Determine instantaneous yield
$$Yield_{Inst} = C_{Inst-Avg} * Q_{PLTs}$$

**S920** — Determine total yield
$$Yield_{Total} = \sum Yield_{Inst}$$

**S922** — $Yield_{Total} > Yield_{Target}$

**S926** — No

Yes

**S924** — End Procedure

# FIG. 9

EUROPEAN SEARCH REPORT

Application Number

EP 24 22 2970

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/368763 A1 (KUSTERS BENJAMIN E [US] ET AL) 26 November 2020 (2020-11-26) | 1,8,9 | INV. A61M1/36 |
| Y | * paragraph [0047] * | 2-7, | B04B5/04 |
| | * paragraph [0054] * | 10-15 | B04B13/00 |
| | * paragraph [0063] * | | |
| | * paragraph [0126] * | | |
| | - - - - - | | |
| Y | US 2009/215602 A1 (MIN KYUNGYOON [US] ET AL) 27 August 2009 (2009-08-27) | 2-7, 10-15 | |
| | * paragraph [0223] * | | |
| | * paragraph [0590] * | | |
| | * paragraph [0592] * | | |
| | * paragraph [0600] * | | |
| | * paragraph [0608] * | | |
| | - - - - - | | |

TECHNICAL FIELDS SEARCHED (IPC)

A61M
B04B

The present search report has been drawn up for all claims

| Place of search | Date of completion of the search | Examiner |
|---|---|---|
| The Hague | 8 April 2025 | Weiss-Schaber, C |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons
............................................................
& : member of the same patent family, corresponding
 document

EPO FORM 1503 03.82 (P04C01)

## ANNEX TO THE EUROPEAN SEARCH REPORT
## ON EUROPEAN PATENT APPLICATION NO.

EP 24 22 2970

This annex lists the patent family members relating to the patent documents cited in the above-mentioned European search report.
The members are as contained in the European Patent Office EDP file on
The European Patent Office is in no way liable for these particulars which are merely given for the purpose of information.

08-04-2025

| Patent document cited in search report | Publication date | Patent family member(s) | | Publication date |
|---|---|---|---|---|
| US 2020368763 A1 | 26-11-2020 | EP | 3741460 A1 | 25-11-2020 |
| | | EP | 4238595 A2 | 06-09-2023 |
| | | US | 2020368763 A1 | 26-11-2020 |
| | | US | 2023011431 A1 | 12-01-2023 |
| US 2009215602 A1 | 27-08-2009 | NONE | | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 101275